# EUROPEAN PATENT SPECIFICATION

(11) **EP 4 125 783 B1**
(45) Date of publication and mention of the grant of the patent: **30.04.2025**
(21) Application number: 21715212.3
(22) Date of filing: 24.03.2021
(51) Int. Cl.: A61Q 1/06, A61K 8/26, A61K 8/9789, A61K 8/02, A61K 8/92

(54) **PIGMENT COMPRISING RAPHANUS SATIVUS EXTRACT AND MONTMORILLONITE**
PIGMENT ENTHALTEND RAPHANUS SATIVUS EXTRAKT UND MONTMORILLONITE
PIGMENT COMPRENANT UN EXTRAIT DE RAPHANUS SATIVUS ET UNE MONTMORILLONITE

(30) Priority: 24.03.2020 GB 202004239; 15.03.2021 GB 202103524
(43) Date of publication of application: 08.02.2023
(73) Proprietor: Givaudan SA, 1214 Vernier (CH)
(72) Inventor: SENNELIER PORTET, Bénédicte, 84450 Jonquerettes (FR); MARTINEZ, Jessy, 84350 Courthezon (FR); CROVILLE, Claire, 84140 Avignon (FR); LAVAUD, Alexis, 68640 Muespach (FR); TARDIEU, Anne-Sophie, 13580 La Fare-les-Oliviers (FR)
(74) Representative: Global Patents
(86) International application number: PCT/EP2021/057509
(87) International publication number: WO 2021/191243

(56) References cited:
- EP-A1- 1 980 239
- WO-A2-2012/033536
- RU-C1- 2 644 178
- GIUSTI M M ET AL: "Anthocyanin pigment compostion of red radish cultivars as potential food colorants", JOURNAL OF FOOD SCIENCE, WILEY-BLACKWELL PUBLISHING, INC, US, vol. 63, no. 2, 1 January 1998 (1998-01-01), pages 219 - 224, XP002191136, ISSN: 0022-1147
- DATABASE GNPD [online] MINTEL; 29 January 2020 (2020-01-29), ANONYMOUS: "Moisturising Colour Lip Tint", XP055815599, retrieved from https://www.gnpd.com/sinatra/recordpage/7219439/ Database accession no. 7219439
- RIBEIRO HÁLISSON L ET AL: "Stabilizing effect of montmorillonite on acerola juice anthocyanins", FOOD CHEMISTRY, ELSEVIER LTD, NL, vol. 245, 22 November 2017 (2017-11-22), pages 966 - 973, XP085314209, ISSN: 0308-8146, DOI: 10.1016/J.FOODCHEM.2017.11.076

## Description

The present invention relates to a pigment for cosmetic applications, in particular for lip coloring cosmetics.

Cosmetics are products used to enhance or change the appearance of the face, fragrance or the texture of the body. They are generally mixtures of chemical compounds derived from natural sources (such as coconut oil), or may be synthetic or artificial. Cosmetics that are applied to the face to enhance one's appearance are also known as make-up, which include items such as lipstick, mascara, eye shadow, foundation, blush, highlighter, bronzer and several other products.

In the United States, the Food and Drug Administration (FDA), which regulates cosmetics, defines cosmetics as "intended to be applied to the human body for cleansing, beautifying, promoting attractiveness, or altering the appearance without affecting the body's structure or functions". This broad definition includes any material intended for use as an ingredient of a cosmetic product.

Color cosmetics or make-up encompasses different categories of products for skin, eyes, cheeks and lips. It is a growing market as consumers increasingly seek to be always selfie-ready. Color cosmetics can enhance overall physical appearance, conceal flaws or define features, making one appear healthier and look more refreshed. Consumers have also expressed that color cosmetics have helped improve their mental well-being, by empowering them and boosting their self-confidence.

The four main categories are: face make-up, such as foundation, blushers, illuminators, face bronzing lotions, creams and powders, loose and pressed powders, and mineral powders; eye make-up, such as eye shadows, eyeliners, eyebrow pencils, kohl and mascara, and mineral powders; lip make-up, such as lipstick, lip glosses, lip pencils, lip plumpers, pots and palettes; and nail make-up, such as nail varnishes and polishes, hardeners and strengtheners, base and top coat.

Lip color is currently the most active color cosmetics category, accounting for 32% of new launches with over 900 million lipsticks sold worldwide every year. Key performance attributes sought by consumers are color (93%), duration or long-lastingness (90%), texture (90%) and finish (89%).

Today, the red pigment used in most of the lipsticks on the market is either synthetic, mineral or derived from crushed cochineal bugs. Most of the existing color formulae rely on synthetic pigments.

There is, however, a growing consumer demand for cleaner, healthier, more natural and more sustainable beauty products. 32% of consumers state that natural / organic make-up is their first choice, and 63% like it but not always buy it. In particular young consumers are looking for vegan alternatives.

Some pigments based on anthocyanins have been described in the prior art, for instance in the following publications:
- WO 2012/033536 A2 relates to red radish and rosemary compositions with enhanced color stability, which may be used in cosmetics, in which the red radish anthocyanins are stabilized by rosmarinic acid.
- EP 1 980 239 A1 relates to cosmetic and dermatological formulations with natural pigments, which may be used in lipstick, lip gloss and other cosmetic products.
- Giusti et al.: "Anthocyanin pigment composition of red radish cultivars as potential food colorants" evaluates red radish cultivars with respect to qualitative and quantitative anthocyanin pigment content.
- "Moisturising Colour Lip Tint" (Mintel Database, accession no. 7219439) relates to a lip balm containing anthocyanins from red radish.
- Ribeiro Halisson et al.: "Stabilizing effect of montmorillonite on acerola juice anthocyanins" investigates the color and anthocyanin stability of clarified acerola juice as affected by different concentrations of montmorillonite.
- RU 2644178 C1 relates to a process for preparing coloring pigments, in which anthocyanins are intercalated with clay.

But consumers are not willing to compromise on performance, even for a 100% natural lipstick. Less than 15% would accept reduced performance with regard to color, coverage and finish. In particular, long-lastingness is clearly an unmet consumer need.

It is therefore an objective of the present invention to provide plant-based pigments of natural-origin, which exhibits excellent color, coverage, long-lastingness and finish characteristics.

This problem has been solved by the pigment and cosmetic composition of the present invention.

In a first aspect, the present invention provides a pigment for use in cosmetic applications, comprising micronized *Raphanus sativus L.* root extract and montmorillonite clay, wherein the micronized *Raphanus sativus L.* root extract has a median particle size (D50) of from about 1.0 µm to about 7.5 µm. The pigment is particularly suitable for application in lip coloring applications.

*Raphanus sativus L.* belongs to the Brassicaceae family. Common names include red radish, garden radish or radish(English), radis (French), or luo bo (transcribed from Chinese). Radishes, the roots of *Raphanus sativus L.* (Radish) and its varieties and cultivars (Cruciferae) are common vegetables with a variety of colors, shapes and sizes. Radishes are annual or biennial brassicaceous crops grown for their swollen tap roots which can be globular, tapering, or cylindrical. The root skin color ranges from white through pink, red, purple, yellow, green to black due to specific pigments called anthocyanins.

Radish is a common vegetable cultivated and consumed all over the world for their roots. In the Western countries, radish is typically known as a small-rooted, short-season vegetable, while in the Far East and Asian countries, a diverse, large-rooted and long-season radish is widely grown. The tap root of radishes is consumed worldwide in the form of pickles, salads and curries due to its high nutritional values. Also, the soft leaves are cooked and used as a leafy vegetable. Radish extracts have also been used to treat stomach disorders, constipation, urinary infections, hepatic inflammation, cardiac disorders and ulcers in folk medicine since the ancient times.

*Raphanus sativus L.* root extract is commercially available, for instance from Yunnan Rainbow Bio-tech. Corp., Ltd. (e.g. "Deodorized Red Radish Extract (Powder)"). A detailed description of this extract is provided in example 1 below. This extract is prepared from the red radish species *Raphanus raphanistrum* subsp. *sativus* (L.) Domin, which is cultivated in China. In mature stage, this red radish species has cylindrical swollen tap roots of about 15 cm length and 3 to 6 cm width. The skin color (internal and external) is purple and the flesh color is pink-purple. An alternative supplier for the *Raphanus sativus L.* root extract is Yunnan Tonghai Yang Natural Products Co., Ltd ("Red radish extract").

Phytochemical studies have demonstrated that red radish root extracts are rich in acylated anthocyanins (Yonghong Liu et al., Stability to Light, Heat, and Hydrogen Peroxide at Different pH Values and DPPH Radical Scavenging Activity of Acylated Anthocyanins from Red Radish Extract. J. Agric. Food Chem. 2007, 55:3692-3701. M. Monica GIUSTI et al., Journal of food science. Characterization of Red Radish Anthocyanins 1996, (61), 2). Acylated anthocyanins have been shown to be a promising alternative to synthetic colorants in food systems (Anna B Kowska-Barczak, Acylated anthocyanins as stable, natural food colorants - a review. Pol. J. Food Nutr. Sci. 2005, 14/55, (2): 107-116).

The authenticity of the botanical raw material used was confirmed by macroscopy, HPTLC fingerprint, HPLC anthocyanins profile, and DNA authentication of the species. The taxonomy (APG III) of the red radish was the following:

| | |
|---|---|
| - Class: | Equisetopsida |
| - Subclass: | Magnoliidae |
| - Superorder: | Rosanae |
| - Order: | Brassicales |
| - Family: | Brassicaceae |
| - Genus: | *Rhaphanus* |
| - Species: | *Raphanus raphanistrum* subsp. *sativus* (L.) Domin |

The extract used in the pigment of the present invention is prepared from the root. These roots are cylindrical swollen tap roots, which are about 15 cm long and 3 - 6 cm wide. The skin color is purple and the flesh is pink-purple.

For the HPTLC fingerprint analysis, a HPTLC plate silica gel 60 F254, 20 x 10 cm was used as the stationary phase, and 1-butanol/water/formic acid 45:15:10 (v/v/v) as the mobile phase. Samples were prepared by suspending 1.0 g of fresh radish root in 10 ml of ethanol (70%), sonicating for 10 min, vortexing, and filtering on 0.45 µm RC. 0.5 µL, 1 µL and 2 µL of the sample, respectively, were applied to the stationary phase and compared to respective amounts of a reference sample. The HPTLC plate was developed with the mobile phase, dried and treated with NP (Natural product reagent) and anisaldehyde. Results were detected at 366 nm and visible light before and after NP-anisaldehyde treatment. Comparison to the reference samples confirmed authenticity. The chromatographic profiles were found to be very similar, with the fresh red radish samples from Yunnan Rainbow having a more intense blue band at R_{f} = 0.9 and a higher anthocyanins content than the reference samples from dried red radish. The test samples are consistent with the fingerprint of a red radish root of *Raphanus raphanistrum* subsp. *sativus* (L.) Domin.

HPLC-DAD chromatography of fresh red radish samples at 520 nm revealed the anthocyanin profile (anthocyanidins and anthocyanosides). Samples from different suppliers were found to be quantitatively similar but different in quantity. Pelargonidin was found to be the major anthocyanidin moiety, and most of the anthocyanosides were esterified with caffeic acid, ferulic acid and maleic acid.

The *Raphanus sativus L.* root extract used in the pigment of the present invention is micronized.

Micronization is the process of reducing the average diameter of a solid material's particles to the micrometer range. Traditional techniques for micronization focus on mechanical means, such as milling and grinding. Modern techniques make use of the properties of supercritical fluids and manipulate the principles of solubility.

Montmorillonite clay is a group of very soft phyllosilicate minerals. It is named after the town Montmorillon in France. Montmorillonite, a member of the smectite group, is a 2:1 clay, meaning that it has two tetrahedral sheets of silica sandwiching a central octahedral sheet of alumina. The particles are plate-shaped with an average diameter around 1 µm and a thickness of 0.96 nm. Individual crystals of montmorillonite clay are not tightly bound hence water can intervene, causing the clay to swell. The water content of montmorillonite is variable and it increases greatly in volume when it absorbs water. Chemically, it is hydrated sodium calcium aluminium magnesium silicate hydroxide (Na,Ca)_{0.33}(Al,Mg)₂(Si₄O₁₀)(OH)₂·nH₂O. Potassium, iron, and other cations are common substitutes, and the exact ratio of cations varies with source.

Montmorillonite clay is a common cosmetic ingredient and is commercially available from various sources. One suitable product is "argile blanche-rosée" (CosWHITE^{™}) supplied by Argile du Velay, France.

The pigment of the present invention has been found to provide excellent color and stability characteristics, while being fully plant-based, edible and of natural origin. This high performance pigment is vegan-compliant. It offers a sustainable alternative to synthetic pigments, while creating new possibilities to answer the growing trend for healthier and cleaner beauty.

The pigment of the present invention comprises anthocyanins. Anthocyanins are water-soluble vacuolar pigments that, depending on their pH, may appear red, purple, blue or black. Food plants rich in anthocyanins include the blueberry, raspberry, black rice, and black soybean, among many others, and of course the red radish used for the pigment of the present invention.

Anthocyanins are known to change their color depending on pH. The typical color scheme going from acidic to basic conditions is red-purple-blue-green. This change in color is caused by protonation/deprotonation, as exemplified here:

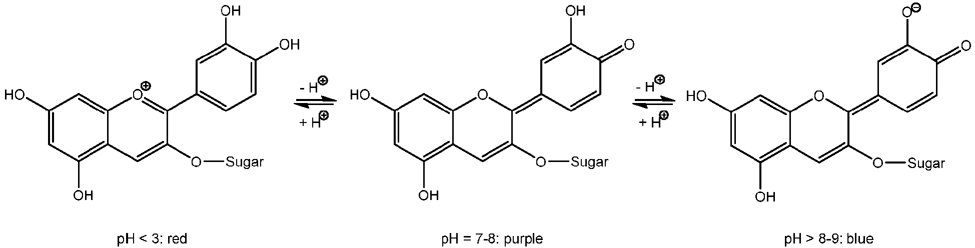

Thanks to these characteristics, it is possible to fine tune the pigment of the present invention for the desired application. In particular, it is possible to adjust the pigment to a particular desired color, for instance red or purple or blue, by adjusting the pH.

For instance, if the pH is adjusted to about 2.8 during the production of the pigment (resulting in a pH of about 3.0 for 1 wt% of pigment in water), a red color (Pantone 1805) is observed; if the pH is adjusted to about 5.5 or 6.5 during the production of the pigment (resulting in a pH of about 6.8 and 7.0, respectively, for 1 wt% of pigment in water), a red-purple color (Pantone 511) is observed; if the pH is adjusted to about 7.5 during the production of the pigment (resulting in a pH of about 7.7 for 1 wt% of pigment in water), a purple color (Pantone 518) is observed; if the pH is adjusted to about 8.5 or 9.5 during the production of the pigment (resulting in a pH of about 9.0 and 9.3, respectively, for 1 wt% of pigment in water), a blue color (Pantone 532) is observed; and if the pH is adjusted to about 11 during the production of the pigment (resulting in a pH of about 10.3 for 1 wt% of pigment in water), a green color (Pantone 451) is observed.

The pH may be adjusted by adding an acid and/or base.

For instance, the pigment of the present invention provides a red color shade corresponding to Pantone 1805 C (L* / a* / b* at 52 / 39 / 18). It has been found to have great performance with regard to shade, intensity and stability. For instance, compared to the synthetic pigment Red 36 Lake, it delivers a better chroma (C* > 60) and higher coverage (Contrast Ratio > 24%) at a concentration of 10 wt%, while providing a shade of red that is akin to the synthetic reference.

For instance, the pigment of the present invention provides a purple color shade corresponding to Pantone 518 C (L* / a* / b* at 42 / 10 / -7.5). It has been found to have great performance with regard to shade, intensity and stability.

Surprisingly, it was found that these colors are stable in lip coloring applications for a pH range from about 3 to about 8. If a higher pH is used, slight changes in color may be observed after application to the lip due to the lower pH of the saliva. Best color stability is observed for a pH range of about 5.5 to about 7.5. In any case, the pH should be kept within the range acceptable for cosmetic applications, and in particular for lip coloring applications.

Therefore, in one embodiment, the present invention provides a red pigment.

In another embodiment, the present invention provides a purple pigment.

Alternatively, the pigment of the present invention may also be pink, blue or green, or any shade in between the previously mentioned colors, for instance red-purple or blue-green.

Throughout this application, the designation "wt%" indicates the weight per weight (w/w) concentration.

The pigment of the present invention also features antioxidant properties, protecting the fragile lip skin from free radicals. Evaluated through different standard tests (ORAC and CAT), and on human epidermal keratinocytes, it showed better antioxidant results than vitamin C, BHT and resveratrol.

The pigment of the present invention allows for a simple formulation in oil, and offers temperature and light stability. Moreover, it has been found to boost the gliding sensation on the lips during the application.

The pigment of the present invention is also safe, Cosmos- and China-compliant, cruelty-free and vegan. In view of the growing consumer demand for cleaner, healthier, more natural and more sustainable beauty products, the pigment of the present invention provides a clean, natural and performant innovation to the most iconic category of color cosmetics: lipsticks, and in particular red lipsticks.

Detailed results on the advantageous performance of the pigment of the present invention are provided in the examples below.

Preferably, the pigment of the present invention comprises micronized *Raphanus raphanistrum* subsp. *sativus* (L.) Domin root extract. This extract has been found to provide the desired red or purple color.

The *Raphanus sativus L.* root extract used in the pigment of the present invention is micronized. It has been found that micronization has a positive impact on chroma and coverage (see example 5 below).

The most commonly used metrics when describing particle size distributions are D-Values: D10, D50 and D90, which are the intercepts for 10%, 50% and 90%, respectively, of the cumulative mass. The D50 is the diameter of the particle that 50% of a sample's mass is smaller than and 50% of a sample's mass is larger than. In other words: D represents the diameter of powder particles, and D50 means a cumulative 50% point of diameter (or 50% pass particle size); D10 means a cumulative 10% point of diameter. D50 is also called average particle size, median diameter, mass-median-diameter (MMD) or log-normal distribution mass median diameter.

According to the present invention, the micronized *Raphanus sativus L.* root extract has a median particle size (D50) of from about 1.0 µm to about 7.5 µm. More preferably, the micronized *Raphanus sativus L.* root extract has a median particle size (D50) of from about 1.5 µm to about 6.0 µm, and most preferably from about 2.0 µm to about 5.0 µm.

In an embodiment of the present invention, the micronized *Raphanus sativus L.* root extract has a particle size 90% percentile (D90) of from about 4.0 µm to about 16.0 µm, more preferably of from about 5.0 µm to about 15.0 µm, and most preferably from about 5.0 µm to about 10.0 µm.

In an embodiment of the present invention, the weight ratio of micronized *Raphanus sativus L.* root extract to montmorillonite clay is from about 1:2 to about 2:1, more preferably from about 1:1.5 to about 1:1.

For instance, the concentration of micronized *Raphanus sativus L.* root extract in the pigment can be from about 10 wt% to about 90 wt%, more preferably from about 30 wt% to about 80 wt%, and most preferably from about 50 wt% to about 70 wt%; e.g. about 62.5 wt% of micronized *Raphanus sativus L.* root extract.

For instance, the concentration of the montmorillonite clay in the pigment can be from about 5% to about 80%, more preferably from about 10% to about 50%, and most preferably from about 30% to about 40%; e.g. about 37.5% of montmorillonite clay.

In an embodiment of the present invention, the pigment of the present invention has an acidic pH. It has been found that an acidic pH improves the stability of anthocyanins and anthocyanin derivatives.

Therefore, in an embodiment of the present invention, the pH of the pigment is from about 2.0 to about 4.0, more preferably from about 2.5 to about 3.5, and most preferably about 2.8. This results in a red pigment.

An acidic pH can be obtained, for instance, by including an acid in the pigment.

Therefore, in an embodiment of the present invention, the pigment of the present invention further comprises an acid, more preferably an organic acid. Citric acid has been found to be particularly well suited, as it is of natural origin and has a suitable pH for application in cosmetics.

In another embodiment, the pH of the pigment is from about 7.2 to about 8.0, more preferably from about 7.4 to about 7.8, and most preferably about 7.5. This results in a purple pigment.

A basic pH can be obtained, for instance, by including a base in the pigment.

Therefore, in an embodiment of the present invention, the pigment of the present invention further comprises a base, more preferably a basic salt. Potassium hydroxide has been found to be particularly well suited, but other hydroxide salts may also be used.

By including both an acid and a base in the pigment of the present invention, it is possible to achieve a buffering effect, leading to an improved color stability.

The acid and/or base may be added before or after micronization. Preferably, it is added prior to micronization.

The acid and/or base may be added before or after any drying steps. Preferably, it is added prior to drying.

The pigment of the present invention may further comprise a suitable carrier.

Therefore, in an embodiment of the present invention, the pigment of the present invention further comprises a polysaccharide, more preferably maltodextrin.

In a particular embodiment, the pigment of the present invention comprises from about 10 wt% to about 40 wt% of micronized *Raphanus sativus L.* root extract, from about 20 wt% to about 50 wt% of montmorillonite clay, from about 2 wt% to about 15 wt% of citric acid, and from about 10 wt% to about 40 wt% of maltodextrin; more preferably, it comprises from about 20 wt% to about 30 wt% of micronized *Raphanus sativus L.* root extract, from about 30 wt% to about 40 wt% of montmorillonite clay, from about 5 wt% to about 10 wt% of citric acid, and from about 20 wt% to about 30 wt% of maltodextrin.

The positive effects of micronization have been discussed above.

In an embodiment of the present invention, not only the *Raphanus sativus L.* root extract is micronized, but also one or more of the other components, in particular the solid components. Preferably, most of the solid components are micronized. More preferably, at least all solid components of the pigment are micronized.

In an embodiment of the present invention, the entire pigment is micronized. The micronization can be done either on the separate components, on several partial mixtures, or on the full composition. It is also possible to perform several micronization steps.

In a second aspect, the present invention provides a cosmetic composition comprising the pigment according to the present invention and a cosmetically acceptable excipient.

Preferably, the cosmetic composition comprises the pigment of one or more of the specific embodiments outlined above.

Any excipients commonly used in the preparation of cosmetic preparations for use on the human skin may be employed in the present invention. Suitable excipients include, but are not limited to ingredients that can influence organoleptic properties, dispersability, appearance, and spreadability of the pigment of the present invention. More specifically, they include liquids, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin, and the like.

The cosmetic composition may take any physical form and may be produced in any solid, liquid, or semi-solid form useful for application

In an embodiment, the cosmetically acceptable excipient comprises castor oil. More preferably, the cosmetically acceptable excipient at least essentially consists of castor oil. Castor oil is of natural origin and has been found to provide a good dispersion of the pigment of the present invention (see also example 7 below).

The cosmetic composition of the present invention is typically a color cosmetic or make-up composition. Possible product forms have been mentioned in the introductory section above.

In an embodiment, the cosmetic composition of the present invention is selected from the group consisting of a lipstick, a lip balm, a lip oil, a lip gloss, a lip scrub, a make-up emulsion, a make-up powder, a make-up oily gel, and a soap.

In a preferred embodiment, the cosmetic composition of the present invention is a lipstick composition.

The present invention is further illustrated by means of the following non-limiting examples:

### Example 1: Raphanus sativus L. Root Extract

*Raphanus sativus L.* root extract is commercially available from several suppliers.

For the present studies, "Deodorized Red Radish Extract (Powder)" was obtained from Yunnan Rainbow Bio-tech. Corp., Ltd. This extract is obtained from red radish of the species *Raphanus raphanistrum* subsp. *sativus* (L.) Domin.

According to the supplier, this commercial extract has the following composition: 42% red radish extract, 42% maltodextrin, 8% citric acid, and 8% water. It is prepared as follows:
- extraction with water
- filtration
- adsorption and desorption with ethanol (70%)
- deodorization
- concentration
- addition of maltodextrin
- addition of citric acid and/or KOH to adjust the pH
- homogeneization
- spray drying
- micronization

The commercial extract is in the form of a red powder.

For the present studies, two different batches of "Deodorized Red Radish Extract (Powder)" from Yunnan Rainbow were used: LT160901 and LT151102.

The anthocyanin content of the two batches was analyzed by HPLC-UV at 520 nm after separation on a C₁₈ column, using perlagonidin-3-O-glucoside as an external standard and applying a molecular weight correction factor (MWCF) after identification of the main compounds with LC-TOF-MS (mainly perlargonidin and anthocyanidin moiety). It was found that LT160901 had an anthocyanin content of 28.72%, and LT151102 of 30.89%.

### Example 2: Red Pigment Preparation

The *Raphanus sativus L.* root extract of example 1 was mixed with montmorillonite clay in a weight ratio of 5:3 in the presence of water. For instance, for a batch of 250 kg of red pigment, 150 kg of water, 60.3 kg of "Deodorized Red Radish Extract (Powder)" and 36.4 kg of montmorillonite clay ("argile blanche-rosée", ref: CosWHITE^{™}, supplied by Argile du Velay, France) were mixed and stirred at room temperature until homogenous. The pH was adjusted to about 2.8 by addition of 3.9 kg of citric acid.

The thus obtained mixture was homogenized by stirring at room temperature and then subjected to spray drying (on a GEA Niro spray dryer) and air jet micronization. Alternatively, it would also be possible to use oven drying, without any influence on the resulting product.

For one particular sample, the particle size distribution was measured before and after the micronization: before micronization D10 = 3.89 µm, D50 = 19.71 µm, D90 = 53.68 µm; after micronization: D10 = 0.66 µm, D50 = 1.91 µm, D90 = 4.19 µm.

The red pigment used for the studies described below was prepared according to the above process and had the following composition:

| **Raw Material** | **Origin** | **INCI** | **Concentration** |
|---|---|---|---|
| *Raphanus sativus L.* root extract | Botanical: *Raphanus sativus L.* | Anthocyanins | 20 - 30 wt% |
| Maltodextrin | Botanical: Zea Mays L. or Manihot esculenta Crantz | Maltodextrin | 20 - 30 wt% |
| Montomorillonite clay | Mineral | Montomorillonite | 30 - 40 wt% |
| Citric acid | Botanical: Zea Mays L. or Manihot esculenta Crantz | Citric acid | 5-10 wt% |

Therefore, the red pigment of the present invention is of 100% natural origin content and 55-70% of natural content according the ISO 16128 assessment.

### Example 3: Characteristics of Red Pigment

The red pigment of example 2 is a red powder. It has a mean particle size (D50) of about 2 µm to about 5 µm.

The anthocyanin content, as determined by HPLC, was from about 12 wt% to about 18 wt%.

The red pigment has a pH of about 2.5 to about 3.5 at 1% in distilled water.

Analysis of the powder by LC-TOF-MS in positive ion mode revealed at least 30 different anthocyanins mainly based on perlargonidin (22), delphinidin (2), cyanidin (5) and malvinidin (1) cores.

Colorimetric analysis provided the following results (E1% is the absorbance at the given wavelength for 1 g of product in 100 ml of solvent):

| L* | a* | b* | C* | h | E1% at 520 nm, pH 3 |
|---|---|---|---|---|---|
| 48.9 | 38.5 | 17.5 | 42.3 | 24.4 | 27.9 |

Thus, the color of the red pigment of the present invention corresponds to Pantone 1805 C.

### Example 4: Stability Studies

### Stability at room temperature

The stability of a first batch of the red pigment of example 3 was tested at room temperature, protected from light and moisture. The results were as follows:

| | Color | | | | Dry matter | Microbiological | |
|---|---|---|---|---|---|---|---|
| | L* | a* | b* | DE2000 | | Total Plate Count [cfu/g] | Yeasts and Moulds [cfu/g] |
| T0 | 49.7 | 38.0 | 17.7 | - | 95.4 wt% | < 100 | < 100 |
| 1 month | 49.1 | 36.9 | 16.7 | 0.85 | 95.2 wt% | < 100 | < 100 |
| 2 months | 50.7 | 38.6 | 17.7 | 1.00 | 96.2 wt% | < 100 | < 100 |
| 3 months | 50.6 | 38.4 | 17.3 | 0.91 | 95.8 wt% | < 100 | < 100 |
| 4 months | 48.9 | 37.3 | 16.9 | 0.87 | 96.1 wt% | < 100 | < 100 |
| 9 months | 49.4 | 36.7 | 15.8 | 1.09 | 94.4 wt% | < 100 | < 100 |
| 12 months | 48.6 | 36.5 | 15.8 | 1.52 | 97.1 wt% | < 100 | < 100 |
| 18 months | 49.3 | 38.4 | 16.9 | 0.95 | 93.3 wt% | < 100 | < 100 |

Thus, the red pigment of the present invention is perfectly stable and complies with the microbiological requirements (Total Plate Count = TPC; Yeasts and Mould = Y & M) for cosmetic ingredients even after 9 months.

### Heat stability

The stability of the first batch of the red pigment of example 3 was also tested at increased temperature:

| | Color | | | | Microbiological | |
|---|---|---|---|---|---|---|
| | L* | a* | b* | DE2000 | TPC [cfu/g] | Y & M [cfu/g] |
| rt | 51.6 | 39.2 | 18.5 | -- | 191 | 0 |
| 30 min at 105 °C | 50.1 | 36.0 | 16.5 | 2.02 | 55 | 0 |
| 30 min at 110 °C | 48.5 | 34.2 | 15.2 | 3.84 | 0 | 0 |
| 30 min at 120 °C | 49.3 | 35.5 | 16.3 | 2.78 | 27 | 0 |

As can be seen from the above, the red pigment of the present invention exhibits good stability even at high temperature. This allows for thermal debacterization if necessary:

| | Color | | | | Microbiological | |
|---|---|---|---|---|---|---|
| | L* | a* | b* | DE2000 | TPC [cfu/g] | Y & M [cfu/g] |
| rt | 51.6 | 39.2 | 18.5 | -- | 191 | 0 |
| 30 min at 120 °C | 43.8 | 34.1 | 14.8 | 2.32 | 0 | 0 |
| 60 min at 120 °C | 50.6 | 37.4 | 16.7 | 1.04 | 0 | 0 |
| 90 min at 120 °C | 49.7 | 35.6 | 15.6 | 1.28 | 0 | 0 |

### Suntest

The color stability of a second batch of the red pigment of example 3 was tested at 450 W/m²:

| | L* | a* | b* | C* | h | DE2000 |
|---|---|---|---|---|---|---|
| T0 | 48.3 | 36.3 | 15.7 | 39.6 | 23.4 | -- |
| 7 h at 450 W/m² | 48.4 | 36.6 | 16.1 | 40.4 | 23.7 | 0.25 |
| 8 h at 450 W/m² | 49.4 | 38.0 | 16.9 | 41.6 | 24 | 1.33 |
| 12 h at 450 W/m² | 49.6 | 37.2 | 16.2 | 40.6 | 23.6 | 1.38 |

Again, the red pigment of the present invention was found to have a good stability.

### Example 5: Impact of Micronization and Montmorillonite Clay

In order to determine the impact of micronization and/or montmorillonite clay, a set of comparative tests was run in a lipstick base containing 10 wt% of red pigment powder.

The following lipstick base was used:

| **Phase** | **Ingredient** | **Concentration** |
|---|---|---|
| A | Butyrospermum parkii butter | 37.5 wt% |
| A | Cera alba | 37.5 wt% |
| A | Caprylic/capric triglycerides | 7.5 wt% |
| B | Castor oil | 17.5 wt% |
| B | Red pigment | 10 wt% |

The following four red pigment powders were tested:
- Comparative sample A: commercial *Raphanus sativus L.* root extract from Yunnan Rainbow as described in example 1
- Comparative sample B: same as comparative sample A, but micronized
- Comparative sample C: red pigment of example 2, but without micronization
- Red pigment of the present invention: red pigment of example 2

Phase A was prepared by blending butyrospermum parkii butter, cera alba and caprylic/capric triglycerides at a temperature of 70 °C until homogeneous. To obtain phase B, a mixture of the respective red pigment powder and the castor oil was homogenized in a turrax blender for 2 min at room temperature. The two phases were combined at 75 °C and blended in the turrax blender for 2 min. The thus obtained lipstick composition was cooled down to room temperature unde magnetic agitation. Finally, it was crushed between glass slabs and applied to a contrast card using a film applicator (film thickness: 50 µm).

L,a,b measurements were performed on a Spectrocolorimeter Konica-Minolta 3600d (D65, L*,a*,b*,h ,C*,Y) and DeltaE(00) was measured to see the color and opacity differences between the different red pigments in lipstick base. The opacity is a measure of covering power, with 100% opacity meaning full coverage. The opacity is calculated as CR (%) = ((Y black*100)/Y white)

The results are shown in the following table:

| | Micronization | Montmorillonite | Hue (h) | Chroma (C*) | CR/opacity (coverage) |
|---|---|---|---|---|---|
| Comparative sample A | no | no | 26.0 | 35.3 | 13.3% |
| Comparative sample B | yes | no | 24.3 | 52.0 | 35.6% |
| Comparative sample C | no | yes | 15.9 | 15.8 | 42.3% |
| Red pigment of the present invention | yes | yes | 29.5 | 60.4 | 30.2% |

As can be seen from the above, micronization leads to an increase in chroma and coverage, while the addition of montmorillonite clay improves the coverage, but decreases chroma and hue.

Best results were observed for the red pigment of the present invention, involving both micronization and montmorillonite clay, which showed a strong increase in chroma, and clear increases for both hue and coverage.

### Example 6: Anti-Oxidant Activity (in vitro)

The anti-oxidant properties of *Raphanus sativus L.* root extract was evaluated using human normal keratinocytes (NHEKs) by quantification of Reactive Oxygen Species (ROS) production after oxidative stress exposure according to the dichlorodihydrofluorescein diacetate (DCFH-DA; "Dichloro-dihydro-fluorescein diacetate (DCFH-DA) assay: a quantitative method for oxidative stress assessment of nanoparticle-treated cells". Aranda et al. Toxicol In Vitro. 2013, 27(2):954-63.) method. In contact with cells, DCFH-DA is cleaved inside the cell to DCFH which is able to interact with reactive oxygen species (ROS) during oxidative stress leading to fluorescence emission. Consequently, the fluorescence emitted is correlated to the antioxidant property of the tested compound.

### Cell culture and pre-treatment

The cell culture was done on primary cells freshly isolated from biopsies.

Normal Human Epidermal Keratinocytes (NHEKs) were seeded in a black plate with a glass bottom at 20'000 cells per well in 96-wells plates with a type I collagen pre-coating in quadruplicate. The cells were incubated for 24 hours in complete medium (Epilife medium supplemented with HKGS) at 37 °C with 5% CO₂.

After 24 hours of culture, the cells were treated with Resveratrol (comparative example) or different concentrations of the commercial *Raphanus sativus L.* root extract of example 1 in complete medium, or with just complete medium (control).

Cells were then incubated for another 24 hours at 37 °C, 5% CO₂.

### ROS measurement

After 24 h of incubation, 2',7'-dichlorofluorescin diacetate (DCFH-DA) probe was added to the wells at 50 µM for at least 30-45 minutes at 37 °C.

Cells were then washed two times with PBS buffer and treated with tert-butyl hydroperoxide solution (TBP) at 5 mM in PBS buffer to cause oxidative stress. For comparison, untreated cells were left in PBS buffer.

Finally, the emitted fluorescence was measured in darkness by excitation wavelength at 488 nm and emission wavelength at 525 nm with microplate reader (TECAN).

The study was carried out in duplicate and with n = 4.

### Statistical analysis

A Shapiro-Wilk normality test was performed to evaluate whether the data follows the Gaussian Law. The results did not follow the Gaussian Law.

As a consequence, a non-parametric statistical analysis was performed by Kruskal-Wallis ANOVA followed by Mann Whitney U test.

### Results

The results are summarized in the following table:

| | | Mean relative to TBP condition | Standard Deviation (SD) | Comparison to oxidative stress, no active |
|---|---|---|---|---|
| No oxidative stress (no TBP, no active) | | 22.4% | 6.32% | |
| Oxidative stress (5 mM TBP) | No active | 100% | 12.65% | - |
| | 200 µM Resveratrol | 24.0% | 6.55% | -76%*** |
| | 0.1 % (w/v) *Raphanus sativus L.* root extract | 23.4% | 23.28% | -77%*** |
| | 1.0% (w/v) *Raphanus sativus L.* root extract | 15.8% | 6.04% | -84%*** |

| | | | | |
|---|---|---|---|---|
| *** p < 0.001 | | | | |

It was confirmed that TBP, which was used to induce oxidative stress, significantly increased the amount of reactive oxygen species (ROS) compared to the untreated control (p<0.001).

Treatment with Resveratrol at 200 µM was used as a positive control.

Under the same conditions, *Raphanus sativus L.* root extract was found to significantly reduce ROS production in a dose-dependent manner.

### Example 7: Dispersion in Different Solvents

The dispersion of the red pigment of example 2 at a concentration of 10 wt% was tested in the following solvents:
- Tris(2-octyldodecyl) citrate (PELEMOL^{®} TGC)
- Triisostearyl citrate (PELEMOL^{®} TISC)
- Refined castor oil (Radia 6132)
- Polyglyceryl-3 diisostearate
- Dodecane (Parafol^{®} 12-97)
- Jojoba seed oil
- Caprylic/capric triglyceride
- Propanediol dicaprylate (DUB ZENOAT^{®})
- Isononyl isononanoate (DUB ININ 14031)
- Octyldodecanol (EUTANOL G)
- Isocetyl stearoyl stearate (DUB SSIC)
- Sunflower seed oil

To assess dispersion, microscope images were taken with 10x zoom without polarized light.

It was found that the red pigment was well dispersed in refined castor oil, tris(2-octyldodecyl) citrate, triisostearyl citrate, and polyglyceryl-3 diisostearate. Of these four, only tris(2-octyldodecyl) citrate is fluid, while the other three solvents are viscous. Furthermore, polyglyceryl-3 diisostearate was found to amend the color of the pigment, making it more blueish. The use of refined castor oil is particularly advantageous because it is fully natural.

The use of dodecane, jojoba seed oil, caprylic/capric triglyceride, propanediol dicaprylate, isononyl isononanoate, octyldodecanol, and sunflower seed oil led to a poor dispersion with agglomerates. Dispersion was slightly better for isocetyl stearoyl stearate.

### Example 8: Impact of Micronization in Lipstick Formulation

A first lipstick base formulation was prepared as described in example 5.

The thus obtained lipstick formulations were applied manually on black-and-white contrast cards; and the spreadability, hue, chroma and opacity were measured using a spectrocolorimeter Konica-Minolta 3600d.

The results were as follows:

| | Spreadability | Hue (h) | Chroma (C*) | CR/opacity (coverage) |
|---|---|---|---|---|
| Lipstick formulation with micronized red pigment | Good spreadability | 29.5 | 60.4 | 30.2% |
| Lipstick formulation with non-micronized red pigment | Spreadability not ok; pigments are not fully crushed | 15.9 | 15.8 | 42.3% |

As can be seen from the above, micronization not only significantly improves hue and chroma, but is also very important for spreadability.

### Example 9: Comparison of Carriers

The red pigment of the present invention comprises montmorillonite clay. In preliminary studies, also other carriers have been tested, using the commercial *Raphanus sativus L.* root extract of example 1:
- Sample D: 100 wt% of *Raphanus sativus L.* root extract; non-micronized
- Sample E: 50 wt% of *Raphanus sativus L.* root extract, 20 wt% of cellulose, 30 wt% Alun; non-micronized; heated to 50 °C for 4 h
- Sample F: 50 wt% of *Raphanus sativus L.* root extract, 10 wt% of cellulose, 40 wt% Alun; non-micronized; heated to 50 °C for 4 h
- Sample G: 50 wt% of *Raphanus sativus L.* root extract, 50 wt% of calcium carbonate; non-micronized
- Sample H: 62.5 wt% of *Raphanus sativus L.* root extract, 37.5 wt% of montmorillonite clay; non-micronized
- Sample J: 62.5 wt% of *Raphanus sativus L.* root extract, 37.5 wt% of montmorillonite clay; micronized

The above powder samples were added to the first lipstick base formulation of example 5 using the same incorporation process as described in example 5.

2 g each of the thus obtained lipstick formulations were applied on black-and-white contrast cards using a Byko-drive G automatic film applicator (film thickness: 102 µm). The spreadability, color, hue, chroma and opacity were measured using a spectrocolorimeter Konica-Minolta 3600d.

The results were as follows:

| | Spreadability | L* | a* | b* | Hue (h) | Chroma (C*) | CR/opacity (coverage) |
|---|---|---|---|---|---|---|---|
| Sample D | average | 69.9 | 31.7 | 15.5 | 26.0 | 35.3 | 13.3% |
| Sample E | many pigments not crushed | 62.0 | 41.8 | 16.9 | 22.0 | 45.1 | 16.0% |
| Sample F | many pigments not crushed | 66.1 | 30.8 | 10.6 | 19.0 | 32.6 | 13.5% |
| Sample G | not all pigments crushed | 49.4 | 33.5 | -2.9 | 355.1 | 33.6 | 24.2% |
| Sample H | many pigments not crushed | 45.8 | 15.2 | 4.3 | 15.9 | 15.8 | 42.3% |
| Sample J | good | 44.0 | 52.5 | 29.8 | 29.5 | 60.4 | 30.2% |

### Example 10: Impact of Montmorillonite Clay in Lipstick Formulation

A second lipstick base formulation was prepared using the following ingredients:
- 37.5 wt% butyrospermum parkii butter
- 10 wt% cera alba
- 10 wt% caprylic/capric triglyceride
- 10 wt% dilinoleic acid/propanediol copolymer
- 5 wt% jojoba esters & helianthus annuus seed wax & acacia decurrens flower way & polyglycerin-3
- 17.5 wt% castor oil
- 10 wt% of pigment (with and without montmorillonite clay)

The following two pigments were tested:
a. red pigment according to the present invention, as described in example 2 above
b. Comparative sample B as described in example 5 above, i.e. without montmorillonite clay

The pigments were first mixed at room temperature with the castor oil using a turrax blender. The other ingredients were mixed and heated to 75 °C until the blend became homogeneous. The pigment/castor oil mixture was then added and mixed until homogeneous. The resulting mixtures were cooled down under magnetic stirring.

2 g each of the thus obtained lipstick formulations were applied on black-and-white contrast cards using a Byko-drive G automatic film applicator (film thickness: 102 µm). Again the spreadability, hue, chroma and opacity were measured using a spectrocolorimeter Konica-Minolta 3600d.

The results were as follows:

| | Spreadability | Hue (h) | Chroma (C*) | CR/opacity (coverage) |
|---|---|---|---|---|
| Red pigment a (with montmorillonite clay) | Good spreadability | 29.9 | 62.4 | 29.7% |
| Red pigment b (without montmorillonite clay) | Spreadability slightly less good | 24.3 | 52.0 | 35.6% |

As can be seen from the above, montmorillonite clay has a positive impact both on hue and chroma, and also slightly improves the spreadability.

### Example 11: Home Use Test

In a home use test, 76 female volunteers in France (age 18-45) were asked to use a lipstick comprising 20% of the red pigment of the present invention at least 4 times during a 10-day test period.

The composition of the lipstick was as follows:

| **Tradename** | **INCI Name** | **Concentration** |
|---|---|---|
| Refined castor oil (Radia 6132) | Ricinus Communis (castor) Seed Oil | 16% (w/v) |
| TiO₂ | CI 77491 (TITANIUM DIOXIDE) | 1% (w/v) |
| Red pigment of example 2 | Montmorillonite, Anthocyanins, Citric acid, Maltodextrin | 20% (w/v) |
| Plurol^{®} Diisostearique CG | Polyglyceryl-3 Diisostearate | 3% (w/v) |
| Dextrose | Dextrose | 0.2% (w/v) |
| Octyldodecanol (Eutanol G) | Octyldodecanol | 9.48% (w/v) |
| Coco-caprylate/caprate (Cetiol LC) | Coco-caprylate/caprate | 6.33% (w/v) |
| Kahlresin 5723 | Glyceryl Rosinate, Octyldodecanol | 2% (w/v) |
| Candelilla wax (Kahlwax 2039L; melting point 71 °C) | Euphorbia Cerifera (Candelilla) Wax | 6.89% (w/v) |
| Carnauba wax (Kahlwax 2442L; melting point 84 °C) | Copernicia Cerifera (Carnauba) Wax | 4.3% (w/v) |
| 2225 Phytowax (dropping point 73 °C) | Helianthus Annuus (sunflower) Seed Cera, Olea Europaea (olive) Oil Unsaponifiables, Rhus Verniciflua Peel Cera/Rhus Succedanea Fruit Cera, Shorea Robusta Resin | 1.72% (w/v) |
| C10-18 triglycerides (Lipocire A; dropping point 35-36.5 °C | C10-18 triglycerides | 11.69% (w/v) |
| Karité CP | Butyrospermum Parkii (Shea) Butter | 4.2% (w/v) |
| Cegesoft^{®} VP (fusion point: 45 °C) | Vegetable Oil, Hydrogenated Vegetable Oil, Euphorbia Cerifera (Candelilla) Wax | 5.16% (w/v) |
| Tsubaki oil deodorised olei-protect EC | CAMELLIA JAPONICA SEED OIL, Polyglyceryl-3 Diisostearate, Water, Ascorbic acid | 4.28% (w/v) |
| JA000040 NATROX RO E20 | Helianthus annuus (Sunflower) seed oil, Tocopherol, Rosmarinus officinalis (Rosemary) leaf extract, Sunflower seed oil glycerides | 0.25% (w/v) |
| Ronaflair Boroneige SF-15 | Boron Nitride | 2.5% (w/v) |
| Soothex ISA | Isostearyl Alcohol, Boswellia Serrata Gum | 0.5% (w/v) |
| Fragrance | FRAGRANCE | 0.5% (w/v) |

Feedback from the volunteers was very good:
- 62% of the volunteers liked the lipstick (78% of those aged 18-34)
- 81% of the volunteers found themselves beautiful
- 75% of them felt sexier
- Compared to other products, it was considered superior in terms of texture, perfume and taste
- For 53% of the volunteers, color duration met their expectations (63% of those aged 18-34)
- The color was found to last for 3 to 4 hours

### Example 12: Purple Pigment Preparation

The *Raphanus sativus L.* root extract of example 1 was mixed with montmorillonite clay in a weight ratio of 5:3 in the presence of water. For instance, for a batch of 1 kg of purple pigment, 625 g of "Deodorized Red Radish Extract (Powder)" and 375 g of montmorillonite clay ("argile blanche-rosée", ref: CosWHITE^{™}, supplied by Argile du Velay, France) were solubilized in 900 ml of water. The pH was adjusted to about 7.5 by addition of 85 g of potassium hydroxide.

The thus obtained mixture was homogenized by stirring at room temperature and then subjected to spray drying (on a GEA Niro spray dryer) and air jet micronization. Alternatively, it would also be possible to use oven drying, without any influence on the resulting product.

Therefore, the purple pigment of the present invention is of 100% natural origin content.

### Example 13: Characteristics of Purple Pigment

The purple pigment of example 12 is a purple powder. It has a mean particle size (D50) of about 2 µm to about 5 µm.

The anthocyanin content, as determined by HPLC, was from about 12 wt% to about 18 wt%.

The purple pigment has a pH of about 7.4 to about 7.8 at 1% in distilled water.

Colorimetric analysis provided the following results (at 10% in a lipstick base):

| L* | a* | b* | C* | h |
|---|---|---|---|---|
| 42.3 | 10.2 | -7.7 | 32.0 | 337 |

Thus, the color of the purple pigment of the present invention corresponds to Pantone 518 C.

### Example 14: Stability Studies

### Stability at room temperature

The stability of a first batch of the purple pigment of example 13 in a standard lipstick base was tested at room temperature (20-21 °C), protected from light and moisture. The results were as follows:

| | Color | | | |
|---|---|---|---|---|
| | L* | a* | b* | DE2000 |
| T0 | 32.4 | 6.0 | -5.3 | -- |
| 3 months | 32.4 | 6.4 | -5.2 | 0.42 |

Thus, the purple pigment of the present invention is perfectly stable.

It was also found to comply with the microbiological requirements (Total Plate Count = TPC; Yeasts and Mould = Y & M) for cosmetic ingredients even after 11 months.

### Stability at 45 °C

The stability of a first batch of the purple pigment of example 13 in a standard lipstick base was also tested at 45 °C, protected from light and moisture. The results were as follows:

| | Color | | | |
|---|---|---|---|---|
| | L* | a* | b* | DE2000 |
| T0 | 32.3 | 6.4 | -5.6 | -- |
| 3 months | 32.2 | 6.9 | -5.5 | 0.54 |

### Heat stability

The stability of the first batch of the purple pigment of example 13 was also tested at increased temperature:

| | Color | | | |
|---|---|---|---|---|
| | L* | a* | b* | DE2000 |
| rt | 42.31 | 10.19 | -7.66 | -- |
| 30 min at 120 °C | 42.04 | 10.97 | -8.03 | 0.71 |
| 60 min at 120 °C | 40.58 | 10.12 | -6.1 | 1.91 |
| 90 min at 120 °C | 40.74 | 10.37 | -6.03 | 1.86 |

As can be seen from the above, the purple pigment of the present invention exhibits good stability even at high temperature. This allows for thermal debacterization if necessary.

### Suntest

The color stability of a second batch of the purple pigment of example 13 was tested at 450 W/m²:

| | L* | a* | b* | C* | h | DE2000 |
|---|---|---|---|---|---|---|
| T0 | 42.31 | 10.19 | -7.66 | 12.75 | 323.07 | -- |
| 24 h at 450 W/m² | 42.90 | 9.92 | -6.49 | 11.85 | 326.82 | 1.00 |

Again, the purple pigment of the present invention was found to have a good stability.

## Claims

1. Pigment for use in cosmetic applications, comprising micronized *Raphanus sativus L.* root extract and montmorillonite clay, wherein the micronized *Raphanus sativus L.* root extract has a median particle size (D50) of from 1.0 µm to 7.5 µm.

2. Pigment according to claim 1, comprising micronized *Raphanus raphanistrum* subsp. *sativus* (L.) Domin root extract.

3. Pigment according to claim 1 or 2, wherein the micronized *Raphanus sativus L.* root extract has a median particle size (D50) of from 1.5 µm to 6.0 µm, and more preferably from 2.0 µm to 5.0 µm.

4. Pigment according to any one of claims 1 to 3, wherein the micronized *Raphanus sativus L.* root extract has a particle size 90% percentile (D90) of from 4.0 µm to 16.0 µm. more preferably of from 5.0 µm to 15.0 µm, and most preferably from 5.0 µm to 10.0 µm.

5. Pigment according to any one of claims 1 to 4, wherein the weight ratio of micronized *Raphanus sativus L.* root extract to montmorillonite clay is from 1:2 to 2:1, more preferably from 1:1.5 to 1:1.

6. Pigment according to any one of claims 1 to 5, wherein the pigment has a red, pink, purple, blue or green color.

7. Pigment according to any one of claims 1 to 6, wherein the pH of the pigment is from 2.0 to 4.0 more preferably from 2.5 to 3.5, and most preferably 2.8.

8. Pigment according to any one of claims 1 to 6, wherein the pH of the pigment is from 7.2 to 8.0, more preferably from 7.4 to 7.8, and most preferably 7.5.

9. Pigment according to any one of claims 1 to 8, further comprising an acid, more preferably an organic acid, most preferably citric acid.

10. Pigment according to any one of claims 1 to 9, further comprising a base, more preferably a basic salt, most preferably potassium hydroxide.

11. Pigment according to any one of claims 1 to 10, further comprising a polysaccharide, more preferably maltodextrin.

12. Pigment according to any one of claims 1 to 11, comprising from 10 wt% to 40 wt% of micronized *Raphanus sativus L.* root extract, from 20 wt% to 50 wt% of montmorillonite clay, from 2 wt% to 15 wt% of citric acid, and from 10 wt% to 40 wt% of maltodextrin; more preferably comprising from 20 wt% to 30 wt% of micronized *Raphanus sativus L.* root extract, from 30 wt% to 40 wt% of montmorillonite clay, from wt% to 10 wt% of citric acid, and from 20 wt% to 30 wt% of maltodextrin.

13. Pigment according to any one of claims 1 to 12, wherein at least all solid components of the pigment are micronized.

14. Cosmetic composition comprising the pigment according to any one of claims 1 to 13 and a cosmetically acceptable excipient, in particular a cosmetically acceptable excipient comprising castor oil.

15. Cosmetic composition according to claim 14, which is a make-up composition, and in particular a lipstick composition.

## Patentansprüche

1. Pigment zur Verwendung in kosmetischen Anwendungen, umfassend mikronisierten Wurzelextrakt von *Raphanus sativus L.* und Montmorillonit-Ton, wobei der mikronisierte Wurzelextrakt von *Raphanus sativus L.* eine mediane Partikelgröße (D50) von 1.0 µm bis 7.5 µm aufweist.

2. Pigment nach Anspruch 1, umfassend mikronisierten Wurzelextrakt von *Raphanus raphanistrum* subsp.*sativus* (L.) Domin.

3. Pigment nach Anspruch 1 oder 2, wobei der mikronisierte Wurzelextrakt von *Raphanus sativus L.* eine mediane Partikelgröße (D50) von 1.5 µm bis 6.0 µm und bevorzugter von 2.0 µm bis 5.0 µm aufweist.

4. Pigment nach einem der Ansprüche 1 bis 3, wobei der mikronisierte Wurzelextrakt von *Raphanus sativus L.* ein 90% Perzentil (D90) der Partikelgröße von 4.0 µm bis 16.0 µm, bevorzugter von 5.0 µm bis 15.0 µm und höchst bevorzugt von 5.0 µm bis 10.0 µm aufweist.

5. Pigment nach einem der Ansprüche 1 bis 4, wobei das Gewichtsverhältnis von mikronisiertem Wurzelextrakt von *Raphanus sativus L.* zu Montmorillonit-Ton von 1:2 bis 2:1, bevorzugter von 1:1.5 bis 1:1, beträgt.

6. Pigment nach einem der Ansprüche 1 bis 5, wobei das Pigment eine rote, rosa, violette, blaue oder grüne Farbe aufweist.

7. Pigment nach einem der Ansprüche 1 bis 6, wobei der pH-Wert des Pigments von 2.0 bis 4.0, bevorzugter von 2.5 bis 3.5 und höchst bevorzugt 2.8 beträgt.

8. Pigment nach einem der Ansprüche 1 bis 6, wobei der pH-Wert des Pigments von 7.2 bis 8.0, bevorzugter von 7.4 bis 7.8 und höchst bevorzugt 7.5 beträgt.

9. Pigment nach einem der Ansprüche 1 bis 8, ferner umfassend eine Säure, bevorzugter eine organische Säure, höchst bevorzugt Zitronensäure.

10. Pigment nach einem der Ansprüche 1 bis 9, ferner umfassend eine Base, bevorzugter ein basisches Salz, höchst bevorzugt Kaliumhydroxid.

11. Pigment nach einem der Ansprüche 1 bis 10, ferner umfassend ein Polysaccharid, bevorzugter Maltodextrin.

12. Pigment nach einem der Ansprüche 1 bis 11, umfassend von 10 Gew.-% bis 40 Gew.-% an mikronisiertem Wurzelextrakt von *Raphanus sativus L.,* von 20 Gew.-% bis 50 Gew.-% Montmorillonit-Ton, von 2 Gew.-% bis 15 Gew.-% Zitronensäure und von 10 Gew.-% bis 40 Gew.-% Maltodextrin; bevorzugter umfassend von 20 Gew.-% bis 30 Gew.-% an mikronisiertem Wurzelextrakt von *Raphanus sativus L.,* von 30 Gew.-% bis 40 Gew.-% Montmorillonit-Ton, von 5 Gew.-% bis 10 Gew.-% Zitronensäure und von 20 Gew.-% bis 30 Gew.-% Maltodextrin.

13. Pigment nach einem der Ansprüche 1 bis 12, wobei wenigstens alle festen Komponenten des Pigments mikronisiert sind.

14. Kosmetische Zusammensetzung umfassend das Pigment nach einem der Ansprüche 1 bis 13 und einen kosmetisch annehmbaren Hilfsstoff, insbesondere einen kosmetisch annehmbaren Hilfsstoff umfassend Rizinusöl.

15. Kosmetische Zusammensetzung nach Anspruch 14, die eine Make-up-Zusammensetzung und insbesondere eine Lippenstiftzusammensetzung ist.

## Revendications

1. Pigment pour une utilisation dans des applications cosmétiques, comprenant un extrait micronisé de racine de *Raphanus sativus L.* et de l'argile montmorillonite, dans lequel l'extrait micronisé de racine de *Raphanus sativus L.* a une taille moyenne de particule (D50) de 1.0 µm à 7.5 µm.

2. Pigment selon la revendication 1, comprenant un extrait micronisé de racine de Domine *Raphanus raphanistrum* subsp. *sativus* (L.).

3. Pigment selon la revendication 1 ou 2, dans lequel l'extrait micronisé de racine de *Raphanus sativus L.* a une taille moyenne de particules (D50) de 1.5 µm à 6.0 µm, et plus préférablement de 2.0 µm à 5.0 µm.

4. Pigment selon l'une quelconque des revendications 1 à 3, dans lequel l'extrait micronisé de racine de *Raphanus sativus L.* a une taille de particule de 90% percentile (D90) de 4.0 µm à 16.0 µm, plus préférablement de 5.0 µm à 15.0 µm, et le plus préférablement de 5.0 µm à 10.0 µm.

5. Pigment selon l'une quelconque des revendications 1 à 4, dans lequel le rapport pondéral de l'extrait micronisé de racine de *Raphanus sativus L.* sur l'argile montmorillonite est de 1:2 à 2:1, plus préférablement de 1:1.5 à 1:1.

6. Pigment selon l'une quelconque des revendications 1 à 5, dans lequel le pigment a une couleur rouge, rose, pourpre, bleue ou verte.

7. Pigment selon l'une quelconque des revendications 1 à 6, dans lequel le pH du pigment est de 2.0 à 4.0, plus préférablement de 2.5 à 3.5, et le plus préférablement de 2.8.

8. Pigment selon l'une quelconque des revendications 1 à 6, dans lequel le pH du pigment est de 7.2 à 8.0, plus préférablement de 7.4 à 7.8, et le plus préférablement de 7.5.

9. Pigment selon l'une quelconque des revendications 1 à 8, comprenant en outre un acide, plus préférablement un acide organique, le plus préférablement l'acide citrique.

10. Pigment selon l'une quelconque des revendications 1 à 9, comprenant en outre une base, plus préférablement un sel basique, le plus préférablement l'hydroxyde de potassium.

11. Pigment selon l'une quelconque des revendications 1 à 10, comprenant en outre un polysaccharide, plus préférablement la maltodextrine.

12. Pigment selon l'une quelconque des revendications 1 à 11, comprenant de 10% en poids à 40% en poids d'extrait micronisé de racine de *Raphanus sativus L.,* de 20% en poids à 50% en poids d'argile montmorillonite, de 2% en poids à 15% en poids d'acide citrique, et de 10% en poids à 40% en poids de maltodextrine ; plus préférablement comprenant de 20% en poids à 30% en poids d'extrait micronisé de racine de *Raphanus sativus L.,* de 30% en poids à 40% en poids d'argile montmorillonite, de 5% en poids à 10% en poids d'acide citrique, et de 20% en poids à 30% en poids de maltodextrine.

13. Pigment selon l'une quelconque des revendications 1 à 12, dans lequel au moins tous les composants solides du pigment sont micronisés.

14. Composition cosmétique comprenant le pigment selon l'une quelconque des revendications 1 à 13 et un excipient cosmétiquement acceptable, en particulier un excipient cosmétiquement acceptable comprenant de l'huile de ricin.

15. Composition cosmétique selon la revendication 14, qui est une composition de maquillage, et en particulier une composition de rouge à lèvres.
